# EUROPEAN PATENT SPECIFICATION

(11) **EP 3 121 265 B1**
(45) Date of publication and mention of the grant of the patent: **06.06.2018**
(21) Application number: 15177983.2
(22) Date of filing: 23.07.2015
(51) Int. Cl.: C12N 1/20, C12P 19/04, C12R 1/01

(54) **KOMAGATAEIBACTER RHAETICUS P 1463 PRODUCER OF BACTERIAL CELLULOSE**
CELLULOSE PRODUZIERENDER KOMAGATAEIBACTER RHAETICUS STAMM P 1463
SOUCHE DE KOMAGATAEIBACTER RHAETICUS P 1463 PRODUISANT DE LA CELLULOSE

(43) Date of publication of application: 25.01.2017
(73) Proprietor: Latvijas Universitate, 1586 Riga (LV)
(72) Inventor: Semjonovs, Pavels, 1024 Riga (LV); Ruklisa, Maija, 1058 Riga (LV); Paegle, Longina, 2114 Olaine (LV); Saka, Madara, 3430 Grobina (LV); Treimane, Rita, 2015 Jurmala (LV); Patetko, Arturs, 1058 Riga (LV); Linde, Raimonds, 1046 Riga (LV)
(74) Representative: Fortuna, Jevgenijs

(56) References cited:
- R. A. C. DOS SANTOS ET AL: "Draft Genome Sequence of Komagataeibacter rhaeticus Strain AF1, a High Producer of Cellulose, Isolated from Kombucha Tea", GENOME ANNOUNCEMENTS, vol. 2, no. 4, 24 July 2014 (2014-07-24), pages e00731-14, XP055238876, DOI: 10.1128/genomeA.00731-14
- VALERA MARIA JOSÉ ET AL: "Cellulose production and cellulose synthase gene detection in acetic acid bacteria", APPLIED MICROBIOLOGY AND BIOTECHNOLOGY, SPRINGER, DE, vol. 99, no. 3, 11 November 2014 (2014-11-11), pages 1349-1361, XP035435455, ISSN: 0175-7598, DOI: 10.1007/S00253-014-6198-1 [retrieved on 2014-11-11]
- VU TUAN NGUYEN ET AL: "Characterization of Cellulose Production by a Gluconacetobacter xylinus Strain from Kombucha", CURRENT MICROBIOLOGY, SPRINGER-VERLAG, NE, vol. 57, no. 5, 14 August 2008 (2008-08-14), pages 449-453, XP019655144, ISSN: 1432-0991, DOI: 10.1007/S00284-008-9228-3
- AYDIN YASAR ANDELIB ET AL: "Isolation and characterization of an efficient bacterial cellulose producer strain in agitated culture: Gluconacetobacter hansenii P2A", APPLIED MICROBIOLOGY AND BIOTECHNOLOGY, SPRINGER, DE, vol. 98, no. 3, 5 November 2013 (2013-11-05), pages 1065-1075, XP035328546, ISSN: 0175-7598, DOI: 10.1007/S00253-013-5296-9 [retrieved on 2013-11-05]
- DUTTA DEBASREE ET AL: "Nitrogen-fixing and cellulose-producing Gluconacetobacter kombuchae sp nov., isolated from Kombucha tea", INTERNATIONAL JOURNAL OF SYSTEMATIC AND EVOLUTIONARY MICROBIOLOGY, vol. 57, no. Part 2, February 2007 (2007-02), pages 353-357, XP002752674,

## Description

### Technical Field

The invention relates to microbiology and biotechnology industry and is directed to isolation of strain producing bacterial nanostructured cellulose and increase of its cellulose production rate.

### Background Art

Owing to the bacteria synthesized cellulose (BC) crystallinity, fibers nano dimensions (diameter 10-30 nm), why BC also is denoted as nanocellulose, its relatively high surface area, which provides its excellent water absorption capacity (up to 99% moisture content), and the ability to form very strong and flexible hydrogel etc. properties, BC is provided a wide range of application and demand on the world market. By using specific bacteria cultivation techniques and controlling the BC fibre formation, it is possible to purposefully regulate its three-dimensional network and synthesize BC in the form of geometric structures *(Klemm et al., 2013),* thus to synthesize bone implants, vascular replacements, etc. This kind of application also is possible due to the BC compatibility with human body tissues, as antibodies are not forming against BC and it do not degrade in human body *(Gama et al., 2013).* In addition, a transparent BC gel with good air permeability and a large amount of absorbed water (up to 200 g of water per 1 g cellulose) so obtained can be used in medicine as wound dressings and as a prolonged drug dosing material.

Unique is also the strength of the bacteria synthesized cellulose (twice higher than in plant-derived cellulose). Dry BC strength is equal to steel strength *(Klemm et al., 2011),* thus BC is recognized as the strongest naturally synthesized biological material *(Lee et al*., *2014).* These uncommon properties, including tensile strength, especially considering the fibers nano-dimensions, provides that the BC can be used for excellent quality audio membranes, textiles, electronic paper, etc. materials production.

For BC synthesis most commonly bacteria of *Acetobacteriaceae* family are used, usually bacteria from the genus *Komagataeibacter,* which is reclassified from its previous name *Gluconacetobacter* (*Yamada et al., 2012),* mainly strains of species *K. xylinus* (Vu et al., 2008) and *K*. hansenii and a Gluconacetobacter kombuchae sp nov. strain (Dutta et al., 2007). Also cellulose synthesizing strains *K. rhaeticus* (from apple juice, *Dellaglio et al.,* 2005 and Valera et al., 2014, later also from the Kombucha tea cell culture) are isolated. Recently draft genome sequence of *K. rhaeticus* strain AF1, isolated from Kombucha tea cell culture, was performed *(Santos et al., 2014).* However, there no study is found (?) showing BC synthesis by use of *K. rhaeticus* strains.

Despite the fact that BC is a product with high added value (in the synthesis cheap and freely available substrates could be used), it has a broad range of application in the economy and has great demand on the world market, the BC production is limited due to high costs. One of the reasons is slow cellulose synthesis by bacterial cells (possibly it is limited by the low growth rate of cellulose producing bacteria and low rate of cellulose crystallization by its cells, *Benziman, 1980*). Also attempt to increase of BC synthesis by using genetic engineering techniques is not succeed *(Gama et al., 2013).*

In addition, the BC production in large-scale fermentation equipment is limited by the fact that it is not possible to obtain BC with the necessary physical properties in the medium mixing mode. High quality BC can be synthesized only in static culturing conditions, but this process rises in the price because of the large spatial capacity of the production. From the above it can be concluded that for the present augmentation of BC synthesis continues to be based on new productive strain isolation, the increase of its BC synthesis by targeted controlling bacterial culturing conditions, and the use of cheap substrates.

### Disclosure of the Invention

Strain B17 isolated from Kombucha tea cell culture has been identified as *Komagataeibacter rhaeticus* and deposited in Microbial Strain Collection of Latvia under the number P 1463. The strain is proposed as promising producer of bacterial cellulose (BC).

Strain *K. rhaeticus* P 1463 synthesize cellulose extracellularly, the obtained cellulose is clean, free from impurities (data of Fourier transform infrared (FT-IR) spectroscopy). When culturing of strain P 1463 in *Hestrin and Schramm* (HS) medium with 2% glucose, 4.4 g/L BC under static culture conditions is obtained, exceeding data referred to in the literature where BC was obtained in similar culture conditions (1.89g/L and 2.3 g/L, *Aydin et al., 2014, Rani et al., 2013).*

The capacity of cellulose synthesis by strain P 1463 is considerably higher compared to the capacity of another strain *K. hansenii* B22, which is isolated in laboratory, and, even more, it is much higher than synthesis capacity of the reference strains *K. xylinus* LMG 1522 and *K. hansenii* LMG 1527 (strains were purchased from German Collection of Microorganisms and Cell Cultures DSMZ/LMG-*Deutsche Sammlung von Mikroorganismen und Zellkulturen GmbH, Leibniz* - *Instituten*)*.*

Strain P 1463 on 2% glucose synthesizes BC one and a half times more compared to *K. hansenii* B22, seven - eight times more compared to references strains *K. xylinus* LMG 1522 and *K. hansenii* LMG 1527. BC synthesis, using the strain P 1463 on fructose, is even higher: it is 1.9 times higher than that obtained using *K. hansenii* B22, and respectively 22.0 times and 8.8 times higher than the BC synthesis achieved using the reference strains *K. xylinus* and *K. hansenii.*

By culturing the bacteria of strain P 1463 in apple juice-containing medium with analogous concentration of sugars during five days we reached BC synthesis of 4.8 g/L with Y_{P/S} (BC yield from the amount of the consumed sugar) - 37.0%. By culturing this strain in apple juice-containing medium for 14 days and adding C-substrate in course of the process we obtained BC 9.5 g/L with Y_{P/S} = 37.7% (with B22: 6.98 g/L with Y_{P/S} = 30.7%; similarly, using whey containing medium with strain P 1463 we obtained BC 6.55 g/L with Y_{P/S} = 42.8% (with B22: 5.25 g/L with Y_{P/S} = 30%).

So advantages of strain P 1463 is good C-substrate utilization capability, relatively high BC synthesis productivity and its yield from the consumed quantity of C-substrate (Y_{P/S}, BC/delta-S, %), and, in addition, good physical properties of the synthesized BC. The following experimental results characterize strain P 1463 isolation, properties and BC synthesis potential of strain compared to others above mentioned BC producers. In addition, the degree of polymerization (DP) of the cellulose synthesized by bacteria of the P 1463 strain is in the range between 2508 and 3300, which is close to the maximum value of the bacterial cellulose *(Gama et al., 2013),* comparatively the B22 cellulose PP is in the range between 1996 and 3200, for the reference strains *K. hansenii LMG 1527* and *K*. *xylinus LMG 1522* it is 1779 and 2200 (according to the data referred to in the literature it is even lower: 775 and 920, respectively, *Wesarg et al., 2010*)*.* In addition, the P 1463 synthesized cellulose has very good mechanical properties: tensile strength and length at break are 65.5 MPa and 16.5 km (comparatively B22 these values do not exceed 26 MPa and 15.2 km). Tensile strength and length at break of the cellulose synthesized by reference strains are even lower (*K. hansenii*: 25.7 MPa and 9.8 km, *K. xylinus*: 17.5 MPa and 9.4 km).

### Brief Description of Drawings

Fig. 1 shows the FT-IR spectrum of cellulose synthesized by strain the *K. rhaeticus* P 1463;
Fig. 2 - the homology and phylogenetic tree of *Komagataeibacter rhaeticus,* wherein the proposed strain P 1463 is identified; designated in the figure by ID 14576; according to Cleenwerck et al 2009 IJSEM 59, 1771-1786, *Komagataeibacter kombuchae* (formerly known as *Gluconacetobacter kombuchae*) is considered as later heterotop synonymous *of Komagataeibacter hansenii* (formerly known as *Gluconacetobacter* hansenii).

### Isolation and identification of cellulose synthesizing strains

Isolation of strains of cellulose producing bacteria was performed by inoculation of decimal dilutions of Kombucha tea cell culture in saline on Petri dishes with *Hestrin and Schramm* (HS) agarized medium, having composition (g/L): D-glucose 20.0, peptone 5.0, yeast extract 5.0, MgSO₄.7H₂O 0.5, Na₂HPO₄.12H₂O 2.7, citric acid 1.15 and agar 15.0. pH = 6.3 ± 0.2, supplemented by cycloheximide (0.1 g/L) in order to prevent growth of yeast cells of Kombucha tea culture. Bacteria were cultivated on Petri dishes at a temperature of 28-30°C for 2-3 days. We visually evaluated the plates and were looking for colonies of potential cellulose synthesizing bacteria, which are light, cream-coloured, even, with smooth or wrinkled edge and, unlike cellulose non-synthesizing acetic acid bacteria, do not form brown water-soluble pigment.

Bacteria of the selected cell colonies were re-cultured in flasks or tubes with liquid HS medium under static conditions at a temperature of 30°C. BC synthesising strains were identified by film formation on the surface of the culture solution after 3 days of cultivation. To ensure that the developed gel consistency film is cellulose, at first it was separated from the culture solution by centrifugation (4000 g, 30 min). In order to release the obtained cellulose from the remains of culture medium and cells, at first it was rinsed with distilled water, then 0.5% NaOH solution was added and together with cellulose heated 20 min at a temperature of 90°C. After cooling and removal of alkaline solution cellulose was rinsed with distilled water up to neutral pH of the medium and dried at a temperature of 105°C. The dry mass of cellulose was determined gravimetrically. Based on the data obtained, we chose the best BC producers, which were marked as B17 (which is strain *K. rhaeticus* P 1463) and B22 (which is strain *K. hansenii* B22). Strains were stored in the liquid HS medium with 15% glycerol additive at a temperature of -20°C.

*The principal characteristics of the bacteria* of strains P 1463 *and B22*: they are gram-negative rods (1.0 µm x 2.0-5.0 µm), rigid, coupled in pairs, form chains or as individual cells, do not form spores, produces cellulose, oxidase reaction is negative, catalase - positive, the optimum temperature for the growth of bacteria 28 - 30°C.

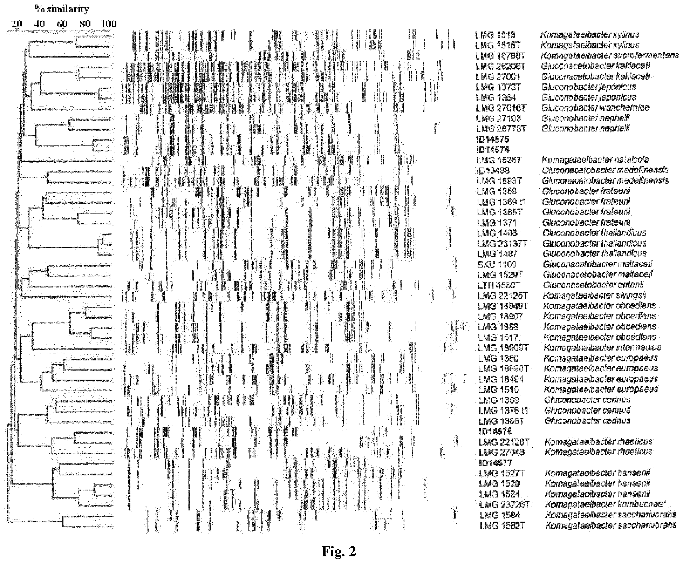

*Strain identification.* It is found that strains B17 (P 1463) and B22 belong to genus *Komagataeibacter* (recently reclassified; its former name Gluconacetobacter, Yamada et al., 2012*).* By identifying up to species it is shown that strain P 1463 (Fig. 2, indicated as ID 14576) is comparable to *K. rhaeticus,* and strain B22 (ID 14577) - to *K. hansenii.* For the identification of genus the method of DNA "fingerprints" (AFLP™) is used; identification up to species is performed by isolation of DNA fragments and their hybridization with DNA-primers (*Cleenwerck et al* 2009).

Strain *K. rhaeticus* B17 is deposited in Microbial Strain Collection of Latvia (MSCL) under the number P 1463. Strain *K. hansenii* B22 is deposited in LUMBI Collection of Microorganisms.

### BC quality characterization by the FT-IR spectroscopy

In order to describe the quality of cellulose (degree of purity, structure), it was prepared for a Fourier transform infrared spectroscopy (FT-IR) analysis. Bacterial cellulose was washed up from medium residues and cells, freeze-dried, then 10 min milled in *Retsch* MM200 ball mill with a frequency of 30 Hz, afterwards the milled cellulose was pressed in tablets together with KBr powder and analyzed using FT-IR spectrometer (model Spectrum One Perkin Elmer, resolution 4 cm⁻¹, 32 repetitions of one analysis of the part of spectrum with a wavelength of 4000-400 cm⁻¹ has been made).

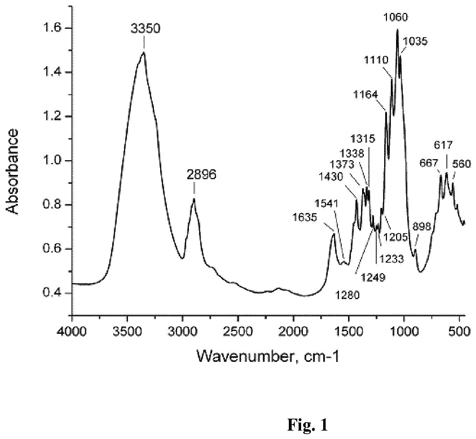

FT-IR analysis of the synthesized cellulose showed a typical cellulose absorption spectrum (peak at a wavelength of 898 cm⁻¹ characterize **β**-1,4-glycosidic bond, Fig. 1), proving, that the synthesized cellulose is clean and free from impurities.

### Assessment of cellulose synthesis capacity of strain P 1463 and B22 (experiments by using inoculum version 1)

For the inoculum cultivation HS culture medium with 2% glucose (hereinafter - inoculum version 1) was used, glucose was sterilized at 0.5 atm, 30 min, the other components of the culture medium - at 1.0 atm, 30 min. Bacteria was cultivated in flasks on a shaker at a temperature of 30°C. After 24 hours cultivation inoculums was added to basic fermentation medium (5% inoculum per fermentation medium volume).

*For the main fermentation* standard HS culture medium or its modifications were used, replacing 2% glucose with 2% fructose or mixture of glucose and fructose (1:1) in a final sugar concentration 2%. BC synthesis was performed under static cultivation conditions, in 300 ml volume flasks with 50 ml medium volume (Low-volume experiment). Parameters (medium pH, gluconic acid and sugar concentrations, the amount of cellulose and its yield from the amount of consumed sugar, Y_{P/S}, %) was analyzed after 3 day (if specified*) and 5 day cultivation of the cell culture.

It was shown, that amounts of cellulose synthesized by strain P 1463, also Y_{P/S} achieved using media with glucose, fructose or mixture of glucose and fructose as carbon sources were significantly higher as compared with those which were obtained by strain B22 in similar culture conditions (Table 1).

**Table 1. Fermentation parameters and BC synthesis by strains P 1463 and B22 under static cultivation conditions using glucose, fructose or its mixture as C-substrate sources (experiments, using inoculum version 1)**

| **Strain** | **S** | **pH** | **Gluconic acid,** g/L | **BC,*** g/L | **BC,** g/L | **Y_{P/S}**, % |
|---|---|---|---|---|---|---|
| **P 1463** | Glucose | 4.3 | 17.00 | 0.40 | **0.85** | 5.90 |
| " | Glucose+fructose | 5.2 | 0.06 | 0.34 | **3.03** | 17.00 |
| " | Fructose | 4.3 | 0.02 | 0.27 | **1.08** | 7.60 |
| **B22** | Glucose | 4.2 | 6.30 | 0.19 | **0.38** | 3.25 |
| " | Glucose+fructose | 5.5 | 1.01 | 0.13 | **0.48** | 6.00 |
| " | Fructose | 6.1 | 0.35 | 0.26 | **0.49** | 6.80 |

### Assessment of cellulose synthesis capacity of strains P 1463 and B22 (experiments, using inoculum version 2)

In this example and all subsequent experiments inoculum was cultivated in modified HS medium, replacing 2% with 1% glucose (inoculum version 2). The main fermentation was performed by culturing bacteria in standard or modified HS medium (replacing: 2% glucose with 4% or 2% glucose with 2% or 4% fructose).

We showed that reduction of glucose concentration in the inoculum medium from 2% (version 1) to 1% (version 2), resulted by a significant increase in BC synthesis: 2.3 (fold?) times using strain P 1463, 4.2 times using strain B22. It resulted also by a significant increase in cellulose yield from consumed sugars (Table 2). Amounts of cellulose synthesized by strain P 1463 in this experiment was 1.94 g/L (or 2.49 g/L, when culture medium was additionally supplemented by FeSO₄ x 2H₂O), were similar or higher compared to BC maximum values obtained by culturing other bacteria in HS medium under static conditions (1.89 g/L, *Aydin et al., 2014*)*.* Amounts of cellulose synthesized by strain P 1463 using medium with 2% or 4% fructose were also comparatively high (1.83 and 2.49 g/L, respectively). It showed that strain P 1463 could be promising BC producer.

We tried to improve cellulose synthesis also by increasing glucose concentration in the main fermentation medium from 2% to 4%. Under these conditions we achieved an increase in glucose consumption by bacterial cells however it was not resulted by an increase in BC production (it even decreased as compared to the control version with 2% glucose) (Table 2). The reason (course?) of low BC production in version with 4% glucose in culture medium was increased synthesis of gluconic acid. Consequently, all subsequent experiments were performed, using the main fermentation medium with the initial sugar concentration 2+/- 0.2%.

**Table 2. Fermentation parameters and BC synthesis when culturing strains P 1463 and B22 in mediums with 2% or 4% glucose or fructose (experiments with inoculum version 2).**

| **Strain** | **S** | **S,** % | **pH** | Δ**S**, g/L | Gluconic acid, g/L | Gluconic acid /Δ**S**, % | Acetic acid, g/L | Acetic acid, /Δ**S**, % | **BC, g/L** | **Y_{P/S}**, % |
|---|---|---|---|---|---|---|---|---|---|---|
| | | | | | | | **0.25** | **1.6** | | |
| **P 1463** | Glucose | **2** | 3.6 | 15,4 | 3.7 | 24.0 | | | **1.94** | **12.6** |
| " | " | **4** | 3.2 | 33,1 | 19.68 | 59.5 | **0.24** | **0.7** | **1.47** | **4.4** |
| | | | | | | | **-** | **-** | | |
| " | *Glucose | **4** | 3.2 | 33,5 | 18.88 | 56.0 | | | **2.08** | **6.2** |
| | | | | | | | **0.30** | **2.5** | | |
| " | Fructose | **2** | 5.4 | 11,9 | 0.15 | 1.3 | | | **1.83** | **15.4** |
| " | " | **4** | 5.5 | 24,6 | 0.27 | 1.1 | **0.33** | **1.3** | **2.49** | **10.1** |
| | | | | | | | **0.26** | **1.6** | | |
| **B22** | Glucose | **2** | 3.7 | 16.0 | 4.98 | 31.0 | | | **1.60** | **10.0** |
| " | " | **4** | 3.1 | 32.9 | 16.93 | 51.5 | **0.25** | **0.8** | **0.99** | **3.0** |
| | | | | | | | **-** | **-** | | |
| " | *Glucose | **4** | 3.4 | 30.9 | 18.88 | 61.0 | | | **0.99** | **3.2** |
| | | | | | | | **0.30** | **2.7** | | |
| " | Fructose | **2** | 6.4 | 11.0 | 0.18 | 1.6 | | | **1.66** | **15.1** |
| " | " | **4** | 5.5 | 20.8 | 0.19 | 0.9 | **0.31** | **1.5** | **1.57** | **7.5** |

| | | | | | | | | | | |
|---|---|---|---|---|---|---|---|---|---|---|
| *FeSO₄ x 2H₂O and deferoxamine (respectively 0,01g/L and 0,003g/L) to the culture medium were additionally supplemented. | | | | | | | | | | |

### BC synthesis by P 1463, B22 and references strains using different sugars as C-sources

A detailed study of sugar utilization capacity and BC synthesis by strain P 1463 (compared to B22 and the reference strains) was performed (Table 3). We showed that P 1463 bacteria have very good potential of glucose, fructose and mannitol (also ribose) utilization and BC synthesis, using these substrates. However, the strain is relatively slow in using sucrose and galactose, consequently the amounts of BC obtained using these substrates are low.

In general utilization potential of glucose, as well as other sugars of all the studied strains is not significantly different. The exception is very low fructose utilization potential of strain *K. xylinus.* In contrast, cellulose synthesis activity of strains differs significantly. Amount of BC synthesized by strain P 1463 in HS medium on glucose was 4.4 g/L, on mixture of glucose and fructose - 4.9 g/L. It's more than under static conditions synthesize strain B22 and much more as synthesize the reference and other strains referred to in the literature *(Aydin et al., 2014, Rani et al., 2013).*

**Table 3. Fermentation parameters and BC synthesis when culturing strains P 1463, B22 and reference strains in mediums with different sugars**

| **Strain** | **S** | **pH** | Δ**S,** g/L | **BC, g/L** | **Y_{P/S}, %** |
|---|---|---|---|---|---|
| **P 1463** | Glucose | 3.6 | 17.4 | **4.40** | **25.3** |
| " | Fructose | 5.1 | 13.5 | **3.34** | **24.7** |
| " | *Glucose + fructose | 5.0 | 10.6 | **3.66** | **34.0** |
| " | Sucrose | 4.5 | 7.7 | **0.47** | **6.1** |
| " | Galactose | 4.5 | 9.6 | **0.79** | **8.2** |
| " | Mannitol | 5.2 | - | **1.74** | **-** |
| " | Ribose | 5.7 | - | **1.18** | **-** |
| **B22** | Glucose | 3.7 | 11.4 | **3.03** | **26.6** |
| " | Fructose | 6.2 | 14.8 | **1.80** | **12.2** |
| " | *Glucose + fructose | 5.3 | 14.3 | **2.26** | **15.8** |
| " | Sucrose | 6.6 | 8.0 | **0.22** | **2.8** |
| " | Galactose | 4.3 | 7.4 | **0.29** | **3.9** |
| " | Mannitol | 5.9 | - | **0.88** | **-** |
| " | Ribose | 5.7 | - | **0.51** | - |
| ***K. xylinus*** | Glucose | | **17.7** | **0.30** | **1.70** |
| " | Fructose | | 5.3 | 0.15 | 2.83 |
| " | Sucrose | | 8.9 | 0.03 | 0.33 |
| " | **Sucrose + fructose | | 4.0+5.0 | 0.51 | 5.67 |
| " | Galactose | | 10.8 | 0.10 | 0.93 |
| " | Mannitol | | - | 0.26 | - |
| " | Ribose | | - | 0.28 | - |
| ***K. hansenii*** | Glucose | | **18.3** | **0.54** | **2.95** |
| " | Fructose | | 10.4 | 0.39 | 3.75 |
| " | Sucrose | | 3.0 | 0.06 | 2.00 |
| " | **Sucrose + fructose | | 7.0 + 3.0 | 0.28 | 2.80 |
| " | Galactose | | 7.9 | 0.13 | 1.64 |
| " | Mannitol | | - | 1.88 | - |

| | | | | | |
|---|---|---|---|---|---|
| *Glucose +fructose (g/L): 20 + 5 g/L | | | | | |

Based on the experimental data shown in Table 4 cellulose synthesis capacity of strain *K*. *rhaeticus* P 1463 on glucose is higher:
1.45 x compared to *K. hansenii* B22;
7.0 x compared to *K. xylinus* LMG 1522;
8.0 x compared to *K. hansenii* LMG 1527.
*Cellulose synthesis capacity of strain P 1463 on fructose is higher:*
1.9 x compared to *K. hansenii* B22;
22.2 x compared to *K. xylinus* LMG 1522;
8.8 x compared to *K. hansenii* LMG 1527.
*BC synthesis using P 1463 on sucrose is low, however it is higher:*
2.0 x compared to *K. hansenii* B22;
9.0 x compared to *K. xylinus* LMG 1522;
6.7 x compared to *K. hansenii* LMG 1527.

**Table 4. BC synthesis by cell culture of P 1463, B22 and the reference strains under static cultivation conditions using glucose, fructose or sucrose as C-substrates.**

| | *Strain* **P 1463** | | *Strain* **B22** | | *Strain **K. xylinus*** | | *Strain **K. hansenii*** | |
|---|---|---|---|---|---|---|---|---|
| ***C-substrate*** | **BC, g/L** | Y_{P/S}, % | **BC, g/L** | Y_{P/S}, % | **BC, g/L** | Y_{P/S}, % | **BC, g/L** | Y_{P/S}, % |
| Glucose | **4.40** | 25.3 | **3.03** | 26.6 | **0.63** | 3.5 | **0.54** | 2.9 |
| Fructose | **3.34** | 27.7 | **1.76** | 11.2 | **0.15** | 2.8 | **0.39** | 3.7 |
| Sucrose | **0.47** | 6.1 | **0.22** | 2.8 | **0.05** | 1.3 | **0.07** | 1.0 |

### Increase of sucrose utilization and BC synthesis by strains in the response to fructose supplementation

Results described in Table 3 shows that all strains are characterized by relatively low sucrose utilization rate and low BC synthesis using this substrate. Further research was aimed to increase sucrose utilization capacity of bacteria, by adding fructose to sucrose-containing medium, (fructose could act as levansucrase inductor and consequently at the same time could improve sucrose utilization by bacterial cells).

Table 5 shows that the addition of fructose significantly improves sucrose utilization by cell culture of strains and BC synthesis. When culturing P 1463 in mediums with sucrose and fructose, in 14 days we obtained BC 3.18 g/L (without fructose 0.74 g/L), when culturing B22 in medium with sucrose and fructose - 3.01 g/L (without fructose 1.43 g/L).

**Table 5. Changes in sucrose utilization and BC synthesis by strains P 1463 and B22 in the response to fructose supplementation to the culture medium**

| **Strain** | **S** | **pH** | **ΔS, g/L** | **BC, g/L** | **Y_{P/S},** % | **Cultivation, h** |
|---|---|---|---|---|---|---|
| **P 1463** | **Glucose** | 3.5 | 17.6 | **2.39** | **13.6** | 24 x 5 |
| " | **Sucrose** | 4.9 | 7.8 | **0.47** | **6.1** | " " |
| " | ***Sucrose+fructose** | 6.1 | 8.9 + 1.4 | **0.47** | **4.5** | " " |
| " | " | 4.4 | 9.1 + 1.9 | **3.18** | **29.0** | 24 x 7 |
| " | **Sucrose** | 6.5 | 7.5 | **0.74** | **9.9** | 24 x 14 |
| " | ***Sucrose+fructose** | 5.0 | 15.0 + 4.4 | **3.14** | **15.5** | " " |
| **B22** | **Glucose** | 3.7 | 17.1 | **1.66** | **9.7** | 24 x 5 |
| " | **Sucrose** | 5.5 | 10.1 | **0.81** | **8.0** | " " |
| " | ***Sucrose+fructose** | 5.3 | 12.8 + 0.7 | **0.76** | **5.6** | " " |
| " | " | 4.4 | 9.8 + 2.2 | **1.79** | **14.9** | 24 x 7 |
| " | **Sucrose** | 5.3 | 18.0 | **1.43** | **7.9** | 24 x 14 |
| " | ***Sucrose+fructose** | 4.7 | 15.0 + 4.5 | **3.01** | **14.7** | " " |

| | | | | | | |
|---|---|---|---|---|---|---|
| * Sucrose + fructose (g/L): 20 + 5 | | | | | | |

### BC synthesis by the strains in apple juice- and whey-containing mediums

Lactose contained in whey before preparation of mediums was hydrolysed by use of enzyme lactase, in result glucose and galactose containing substrate was obtained. Apple juice-, whey- and glucose- (control version) containing culture mediums were prepared on the basis of calculations for initial reducing sugar concentration in medium of all versions being equal to 2%.

It was shown, that utilization rate of sugars by cell culture of strains from apple-juice (glucose, fructose and sucrose) and whey-containing media (glucose) does not differ from glucose utilization in control version. Exception is very low galactose utilization by strains from whey containing media.

Culturing P 1463 bacteria in apple juice-containing mediums BC 4.77 g/L was obtained, in whey containing mediums - 2.86 g/L. Slightly smaller quantities of BC on these substrates were obtained by culturing bacteria B22. Increase of BC synthesis (also Y_{P/S}) culturing bacteria in apple juice- and whey-containing mediums compared to glucose-containing mediums could be explained with improved medium buffer-capacity and pH regulation using these substrates.

**Table 6. Sugar utilization and BC synthesis by strains P 1463 and B22 in glucose (control), apple juice- or whey-containing media (Low-volume experiments, 5 days)**

| **Strain** | **C-substrate source** | **pH** | **ΔS, g/L** | **BC, g/L** | **Y_{P/S},** % |
|---|---|---|---|---|---|
| P 1463 | **Glucose** | 3.8 | 17.3 | **2.54** | 14.7 |
| " | Apple juice | 4.5 | 12.9 | **4.77** | 37.0 |
| " | Whey | 4.2 | 11.4 | **2.86** | 25.1 |
| **B22** | Glucose | 3.9 | 16.1 | **2.36** | 14.7 |
| " | Apple juice | 5.6 | 15.3 | **3.50** | 22.9 |
| " | Whey | 4.0 | 12.9 | **2.90** | 22.5 |

### BC synthesis in mediums with glucose, sucrose, apple juice and whey (Large volume experiment)

Large volume experiments were performed by culturing bacteria in 3000 ml glass jars with 200 ml culture medium. As C-substrates for bacteria cultivation glucose, sucrose apple juice and whey were used. Culturing bacteria for 14 days and gradually adding C-substrate during the cultivation process, amount of utilized C-substrate, as well as of the synthesized cellulose increased (Table 7).

Culturing P 1463 bacteria in apple juice-containing mediums, amount of synthesized BC reached 9.49 g/L, in whey-containing medium - 6.55 g/L (comparatively in mediums with glucose 8.09 g/L). Culturing B22 in these mediums, amount of synthesized BC was significantly lower, respectively 6.98, 5.25 and 6.29 g/L. Similarly as in experiments previously described, culturing strains in sucrose-containing mediums amount of synthesized cellulose was relatively low (3.47 and 2.41 g/L). This example with a higher reliability confirmed advantages of strain P 1463, as well as, that in BC synthesis cheap sugars-containing raw materials can be used, and by performing graduall adding of C-substrate during the process of fermentation, relatively large amounts of BC could be obtained.

**Table 7. Substrate utilization and BC synthesis by strains P 1463 and B22 using glucose, sucrose, apple juice and whey as C-substrate sources (Large volume experiment, 14 days)**

| **Strain** | **C-substrates** | **pH** | **ΔS, g/L** | **BC, wet weight g/L** | **BC, dry weight, g/L** | **Y_{P/S}**, % |
|---|---|---|---|---|---|---|
| **P1463** | **Glucose** | 4.3 | 24.75 | **346.0** | **8.09** | 32.69 |
| " | **Sucrose** | 4.4 | 25.78 | **338.4** | **3.47** | 13.46 |
| " | **Apple juice** | 5.4 | 25.15 | **320.0** | **9.49** | 37.73 |
| " | Whey | 5.5 | 15.31 | **216.2** | **6.55** | 42.78 |
| **B22** | **Glucose** | 3.8 | 21.82 | **348.0** | **6.29** | 28.82 |
| " | **Sucrose** | 4.6 | 22.68 | **223.0** | **2.41** | 10.63 |
| " | **Apple juice** | 4.8 | 22.72 | **420.0** | **6.98** | 30.72 |
| " | **Whey** | 4.7 | 17.13 | **268.0** | **5.25** | 30.65 |

### Properties of BC synthesized by P 1463, B22 and the reference strains

Experiments, the duration of which were 14 and 40 days (Table 8) showed that the use of strain P 1463 can results in synthesis of cellulose with degree of polymerization (DP) in the range between 2508 and 3300, which is close to the maximum DP of bacterial cellulose referred to in the literature *(Gama et al., 2013).* DP of B22 cellulose was in the range between 1500 and 3200, DP of the reference strains *K. hansenii* LMG 1527 and *K. xylinus* LMG 1522 it was, respectively 1779 and 2200. Values of DP of the latter strains referred to in the literature are even lower, i.e. 775 to 920 (*Wesarg et al., 2010*)*.* In addition, the P 1463 synthesized cellulose had very good mechanical properties: tensile strength and lenght et break, respectively reached 65.5 MPa and 16.5 km (comparatively these values for B22 do not exceed 26 MPa and 15,2 km). Tensile strength and lenght at break of cellulose synthesized by reference strains **were** even lower (*K. hansenii*: 25.7 MPa and 9.8 km, *K. xylinus*: 17.5 MPa and 9.4 km).

**Table 8. Degree of polymerization and mechanical strength of BC synthesized by P 1463, B22 and the reference strains**

| **Strain** | **Cultivation,** days | **DP** | **BC tensile strength,** MPa | **BC lenght at break,** km | **BC stretch ratio,** % |
|---|---|---|---|---|---|
| **B17** | 14 | 2508 | 25.7 | 12.3 | - |
| " | 40 | **3300** | **65.5** | **16.5** | **3.9** |
| B22 | 14 | 1996 | 15.6 | 12.4 | - |
| " | 40 | 3200 | 19.6 | 15.2 | 3.7 |
| *K. xylinus* | 40 | 2200 | 17.5 | 9.4 | 1.9 |
| *K.hansenii* | 40 | 1779 | 25.6 | 9.8 | 2.6 |

These examples show that *K. rhaeticus* P 1463 is an outstanding BC producer: in static culture conditions bacteria of this strain from a variety of sugars synthesized significantly greater quantities of BC compared to our isolated strain *K. hansenii* B22 and in particular the cellulose quantities synthesized by the reference strains *K. xylinus* LMG 1522 and *K. hansenii* LMG 1527. In addition, cellulose synthesized by *K. rhaeticus* B17 have much better mechanical properties.

### Sources of Information

1. Aydin, Y., Aksoy, N. Isolation and characterization of an efficient bacterial cellulose producer strain in agitated culture: Gluconacetobacter hansenii P2A. (2014) Appl Microbiol Biotechnol, 98: 1065 - 1075.
2. Benziman, M., Haigler, C. H., Brown, M. R., White, A. R., Cooper, K. M. (1980) Cellulose biogenesis: polymerization and crystallization are coupled processes. In: Acetobacter xylinum. Proc Natl Acad Sci USA 77: 6678-6682.
3. Czaja, W., Krystynowicz, A., Bielecki, S., Brown, R.M. Jr. (2006) Microbial cellulose- The natural power to heal wounds. Biomaterials 27: 145-151.
4. Dellagio, F., Cleenwerck, I., Felis, G., Engelbeen, K., Janssens, D., Marzotto, M.(2005) Description of Gluconacetobacter swingsii sp. nov. and Gluconacetobacter rhaeticus sp. nov., isolated from Italian apple fruit. Int J Systematic and Evolutionary Microbiol, 55:2365-2370.
5. Gama, M., Gatenholm, P., Klemm, D. (2013) Bacterial nanocellulose. A sophistical multifunctional material. CRC press, Boca Raton, London, New York, 272 pp.
6. Klemm, D., Ahrem, H., Kramer, F., Fried, W., Wippermann, J., Kinne, R.W. (2013) Bacterial nanocellulose hydrogels designed as bioartificial medical implants. In: Bacterial nanocellulose. A sophistical multifunctional material, CRC press, Tokyo and French group, Boca Raton, London, New York, pp.175-196.
7. Klemm, D., Kramer, F., Moritz, S., Lindstroem, T., Ankerfors, M., Gray, D., Dorris, A. (2011) Nanocellulose: a new family of nature-based materials. Angew Chem. Int.Ed. 50:5438-5466.
8. Lee, K.Y., Buldum, G., Mantalaris, A., Bismarck, A. (2014) More than meets the eye in bacterial cellulose: biosynthesis, bioprocessing and applications in advanced fiber composites. Macromol Biosci 14:10-32.
9. Rani, M. U., Appaiah, K. A. A. (2013) Production of bacterial cellulose by Gluconacetobacter hansenii UAC09 using coffee cherry husk. J Food Sci Technol 50 (4):755-762.
10. Santos, R. A. C., Berretta, A. A., Barud, H., Ribeiro, S. J. L., Gonzalez-Garcia, L. N., Zucchi, T. D., Goldman, G. H., Riano-Pachon, D. M. (2014) Draft genome sequence of Komagataeibacter rhaeticus strain AF1, a high producer of cellulose, isolated from Kombucha tea. Genome Annoucements, 2, 4, e00731-14.
11. Wesarg, F., Hefler, N., Kralisch, D., Klemm, D., Fried, W., Muller, F.A.(2010) Structural and mechanical properties of bacterial nanocellulose produced by different Gluconacetobacter strains. Abstr. 239th American Society National, San Francisko, CA.
12. Yamada, Y., Yukphan, P., Vu, H., Muramatsu, Y., Ochaikul, D., Tanasupawat, S., Nakagawa, Y. (2012) Description of Komagataeibacter gen. nov., with proposals of new combinations (Acetobacteraceae). J Gen Appl Microbiol, 58: 397 -404.
13. Valera M.J. et. al (2014) Cellulose production and cellulose synthase gene detection in acetic acid bacteria. Applied Microbiology And Biotechnology, vol. 99, No. 3, page 1349-1361.
14. Vu T.N et.al (2008) Characterization of Cellulose Production by a Gluconacetobacter xylinus Strain from Kombucha. Current Microbiology, vol. 57, No. 5, pages 449-453.
15. Dutta D. et. al (2007) Nitrogen-fixing and cellulose-producing Gluconacetobacter kombuchae sp nov., isolated from Kombucha tea. International Journal Of Systematic And Evolutionary Microbiology, vol. 57, Part 2, pages 353-357.

## Claims

1. Bacterial cellulose synthesizing strain *Komagataeibacter rhaeticus,* which is deposited in Microbial Strain Collection of Latvia under the number P 1463.

2. Use of the strain *Komagataeibacter rhaeticus* P 1463 according to claim 1 for cellulose synthesis.

## Patentansprüche

1. Bakterieller zellulose-synthetisierender Stamm *Komagataeibacter rhaeticus,* der in der Mikroben- Stammsammlung von Lettland unter der Nummer P 1463 gelagert wird.

2. Verwendung des Stammes *Komagataeibacter rhaeticus* P 1463 gemäß Anspruch 1 für die Zellulosesynthese.

## Revendications

1. Souche de synthèse de cellulose bactérienne *Komagataeibacter rhaeticus,* qui est déposée dans la Collection de souches microbiennes de Lettonie sous le numéro P 1463.

2. Utilisation de la souche *Komagataeibacter rhaeticus* P 1463 selon la revendication 1 pour la synthèse de cellulose.
